## Europäisches Patentamt

## European Patent Office

## Office européen des brevets

(11) Publication number: **0 029 727**
**A2**

(12) **EUROPEAN PATENT APPLICATION**

(21) Application number: **80304198.7**

(22) Date of filing: **24.11.80**

(51) Int. Cl.³: **A 61 L 15/07**
**A 61 F 13/04**

(30) Priority: **27.11.79 GB 7940960**
**04.09.80 GB 8028624**

(43) Date of publication of application:
**03.06.81 Bulletin 81/22**

(84) Designated Contracting States:
**AT BE CH DE FR GB IT LI LU NL SE**

(71) Applicant: **D. Byford & Company Limited**
**P.O. Box 63 Abbey Lane**
**Leicester LE4 ODX(GB)**

(72) Inventor: **Sirisena, Sarath De Alwis**
**12 Woodhall Close**
**Leicester(GB)**

(74) Representative: **SERJEANTS**
**25, The Crescent**
**Leicester, LE1 6RX(GB)**

(54) Fabrics for surgical splints or casts and methods for stiffening such fabrics.

(57) The invention provides a fabric combination for surgical splints or casts including (A) a first material for forming a first inner sleeve with a moisture absorbing fibrous component and a component having a wicking action, and (B) a second material for forming a second outer sleeve having a solvent dissolvable component and a reinforcing component for stiffening the outer sleeve when the solvent dissolvable component has been subjected to solvent action and has congealed against the reinforcing component.

EP 0 029 727 A2

-1-

TITLE:

Fabrics for surgical splints or casts and methods for
stiffening such fabrics

DESCRIPTION

Field of invention

The invention relates to fabrics for surgical splints
or casts and to the methods for stiffening such fabrics.

Background of the invention

Surgical splints or casts have been made from flexible
fabric containing constructions which are hardened by an
appropriate reaction brought about by irradiation,
cooling or treatment with a chemical substance for
example.

The British Patent Specification 600,575; British Patent
Specification 704,478 and British Patent Specification
709,130 discloses fabrics in which solvents are applied
to harden a fabric construction. Cellulose acetate, also
known as diacetate, could be used as the fibre and acetone as solvent. The British Patent Specification 1,413,047 describes a material containing in addition glass fibre.

The British Patent Specification 1,335,123 uses a combination of an immobilizing outer sleeve and an inner protective sleeve. The outer sleeve is curved to make it rigid by irradiation.

Materials containing diacetate have generally been hardened by exposure to acetone optionally incorporating some retardant.

It is the purpose of the invention to provide fabrics for surgical splints or casts which can be made rigid with a solvent and are comfortable in use.

It is a further purpose to provide fabrics which can be formed into a hard cast or splint in situ on the patients body.

It is yet a further object of the invention for providing a method for forming splint and casts with reduced fire risk and reduced exposure by patients and nursing staff to solvent.

Summary of Invention

According to the invention there is firstly provided a fabric combination for surgical splints or casts including (A) a first material for a first inner sleeve with a moisture absorbing fibrous component and a component having a wicking action, and (B) a second material for a second outer sleeve having a solvent dissolvable component and a reinforcing component for stiffening the outer sleeve when the solvent dissolvable component has been subjected to solvent action and has congealed against the reinforcing component. The first sleeve in a splint protects the patients skin whilst the second sleeve is subjected to solvent action. The first sleeve also provides a comfortable layer next to the patients skin in use which will not be saturated unduly with moisture. The second sleeve can be made rigid in situ on the patients limb.

Moisture absorbing fibrous components are generally natural fibres such as cotton, linen, wool and man-made equivalents or derivatives such as viscose. Moisture absorbing fibres generally have a comfortable feel. Components having a wicking action include fibres which are hollow or have accessible capillaries. Preferably such components should have a low moisture absorption so as to avoid them absorbing and retaining undesired moisture. Wicking action components may be modacrylics or polyolefin derived fibres.

Preferably the first material includes separate cotton and polypropylene yarns. The polypropylene yarn displays a good wicking action. Alternatively Dunova (Registered Trade Mark) made by Bayer or Viloft (Registered Trade Mark) made by Courtaulds can be used. Preferably the first material is in the form of an elastic fabric so that the first sleeve will readily conform to the shape of the patients skin. Advantageously the first sleeve is in the form of a rib-knitted tubular fabric which can be stretched and slipped around a patients limb before stiffening.

Preferably the second material is in the form of an elastic tubular fabric and contains from 0.5 to 5% of an elastomer fibre. This ensures a close fitting of the second sleeve around the first and good support. Preferably the second sleeve is in the form of a rib knitted tubular fabric which can be slipped around the patients limb at the same time and together with the first sleeve. The rib structure provides a good fabric thickness, giving a high strength to the splint or cast. Preferably the second material comprising separate solvent dissolvable and reinforcing yarns is incorporated into a single rib knitted tubular fabric by plating. The plated structure gives good solvent access to the diacetate yarn yet ensures firming binding of the glass fibre to the dissolved and subsequently solidified diacetate in the stitches of the fabric. Preferably cellulose diacetate is the dissolvable component and the second sleeve includes glass-fibre reinforcing component. Other appropriate combinations of dissolvable and reinforcing yarns may be used with the first sleeve as described previously. It has been found that suitably the second material should include from 40 to 60% by weight of the cellulose diacetate for the best strength of the cast and simultaneous air permeability.

The fabrics may be dyed or heated in any way desired where required heel and/or toe pouches can be provided.

The material for forming the sleeves of tubular knitted material can be slipped over a limb. Advantageously however the first and second materials are in the form of non-tubular fabric pieces having fastening means at edges to form the first and second sleeve when said edges are mutually fastened together. The materials may be made into fabric pieces by cutting tubular knitted material open. After the cast or splint has stiffened, it may be slipped off by undoing the fastening means. The fastening means may be a zip, Velcro (Registered Trade Mark) type fastener or hook or button devices as desired. These materials have the additional advantage that they may be applied without sliding them over the patients limb. The fastening means also hold the first and second materials together.

Preferably a third material for forming a sleeve interposed between the first and second sleeve, which third material is impervious to solvent used for stiffening the outer sleeve but soluble in water. Thus the patients skin can be shielded against the solvent applied for stiffening purpose. Yet on washing, the third material will dissolve and enable moisture to pass from the patients skin to surrounding air. Conveniently the third layer is a thin sheet of polyvinyl alcohol soluble at room temperature.

Diacetate yarns can be dissolved by acetone. This solvent is flammable. Conveniently therefore the splint or cast is applied by a method which comprises dipping at least the first and second material in a solvent including consisting predominantly of dichloromethane, forming the materials into substantially tubular sleeves, and evaporating the solvent from the sleeves whilst the sleeves are retained in a nitrile rubber cover for absorbing the solvent evaporated from the sleeves. The dichloromethane is thus applied to the material without much escape of solvent to atmosphere. This benefits both patient and nursing staff. The nitrile rubber can also absorb a large quantity of dichloromethne and can be re-used after "drying".

Preferably the solvent includes from 1 to 20% by weight of 1.1.1 trichloroethane as a retarder. More than 20% of 1.1.1 trichloroethane prevents stiffening of the splint or cast. Accordingly the invention also provides a solvent comprising from 80 to 99% by weight of dichloromethane and for the balance substantially of 1.1.1 trichloroethane.

### Example 1

A first fabric is knitted on a double cylinder machine of 3¼ inch diameter and having 84 needles. A cotton yarn (200 decitex) and polypropylene filament yarn (320 decitex) are fed in plating relationship at each feed and the needles are controlled to give a 1x1 rib tubular sleeve.

A second fabric can be knitted on a similar machine from cellulose diacetate (230 decitex), glass fibre (350 decitex). At one or more of the knitting stations

a 0.4 decitex Lycra elastomer yarn is fed in addition to the cellulose diacetate and the glass fibre. 1x1 rib tubular sleeve results.

The fabrics can be sold together in a pack or supplied separately.

In use the first fabric is slipped over a broken limb to form an inner sleeve. Any pressure pads can then be placed over it. Subsequently a second fabric is pulled as an outer sleeve over the first fabric. Acetone is sprayed over the outer sleeve. The cellulose diacetate becomes fluid and spreads against the glass fibre yarn. The acetone evaporates. The diacetate becomes a binder for a frame work of the glass fibre yarn which is thus held rigid. The inner sleeve guards the patients skin against exposure to acetone. Any acetone which penetrates the inner sleeve evaporates. The final structure of the outer sleeve, while rigid, still has openings through which evaporation from the inner sleeve can take place. If extra strength is required more than one outer sleeve can be applied in the manner described.

The moisture from the patients skin is absorbed and conducted away towards the outer sleeve by the combined effect of the polypropylene and cotton to enable the moisture to evaporate and the patients skin to be kept dry.

In this way a cast or splint can be formed which is both comfortable, light, and strong. Other advantages are considerable fire resistance, the ability to colour or dye the yarn used, water resistance, X-ray permeability and the adaptation of the strength which is possible. The splint or cast can be applied easily and removed by applying more acetone.

CLAIMS:

1. A fabric combination for surgical splints or casts having a first material for forming a sleeve having a solvent dissolvable component and a reinforcing component for stiffening the sleeve when the solvent dissolvable component has been subjected to solvent action and has congealed against the reinforcing component, characterised in that a second material is provided for forming an inner sleeve within the aforementioned sleeve of the first material, said second material including a moisture absorbing fibrous component and a component having a wicking action.

2. A fabric combination according to claim 1 further characterised in that the second material includes a cellulosic fibrous component having good moisture absorption and a hydrophobic wicking action component.

3. A fabric combination according to claim 1 or claim 2 further characterised in that the second material includes separate cotton and polypropylene yarns.

4. A fabric combination according to any of the preceding claims further characterised in that the

-11-

second material is in the form of an elastic fabric.

5. A fabric combination according to claim 4 further characterised in that the second material has a rib--knitted fabric structure, the moisture absorbing component and the component having a wicking action being of separate yarns combined by plating.

6. A fabric combination according to any of the preceding claims further characterised in that the first material is in the form of an elastic tubular fabric for conforming to the outside face of the second material.

7. A fabric combination according to claim 6 further characterised in that the first material contains from 0.5 to 5% of an elastomer fibre and is in the form of a rib knitted tubular fabric.

8. A fabric combination according to claim 7 further characterised in that the first material comprising separate solvent dissolvable and reinforcing yarns is incorporated into a single rib knitted fabric by plating.

9. A fabric combination according to any of the preceding claims further characterised in that the first material includes cellulose diacetate as the dissolvable component and glass-fibre reinforcing component.

10. A fabric combination according to claim 9 further characterised in that the first material includes from 40 to 60% by weight of the cellulose diacetate and from 60 to 40% by weight of the glass fibre.

11. A fabric combination according to claim 1 further characterised in that the first and second materials are in the form of non-tubular fabric pieces having fastening means at edges to form the first and second sleeve when said edges are mutually fastened together.

12. A fabric combination according to any of the preceding claims further characterised in that a third material for forming a sleeve is provided for interposing between the first and second material, which third material is impervious to solvent used for stiffening the sleeve of the first material but insoluble in water.

13. A method for forming a surgical splint from a fabric combination according to any of the preceding claims including cellulose diacetate as the dissolvable component which comprises dipping at least the first and second material in a solvent consisting predominantly of a halogenated hydrocarbon, forming the materials into substantially tubular sleeves, and evaporating the solvent from the sleeves whilst the sleeves are retained in a nitrile rubber cover for absorbing the solvent evaporated from the sleeves.

14.   A method according to claim 13 in which the solvent includes from 1 to 20% by weight of 1.1.1 trichloroethane as a retarder.

15.   A solvent comprising from 80 to 99% by weight of dichloromethane and for the balance substantially of 1.1.1 trichloroethane for use in a method according to claims 13 or 14.